# EUROPÄISCHE PATENTSCHRIFT

(11) **EP 2 297 108 B1**
(45) Veröffentlichungstag und Bekanntmachung des Hinweises auf die Patenterteilung: **08.02.2012**
(21) Anmeldenummer: 09749877.8
(22) Anmeldetag: 20.05.2009
(51) Int. Cl.: C07D 221/18, C09B 3/16

(54) **CHINOIDE RYLENDICARBOXIMIDE ALS IR-ABSORBER**
QUINOID RYLENE DICARBOXIMIDES AS IR ABSORBERS
RYLÈNEDICARBOXIMIDES QUINOÏDES EN TANT QU'ABSORBEURS DE RAYONS INFRAROUGES

(30) Priorität: 23.05.2008 EP 08156849
(43) Veröffentlichungstag der Anmeldung: 23.03.2011
(73) Patentinhaber: BASF SE, 67056 Ludwigshafen (DE); Max-Planck-Gesellschaft zur Förderung der Wissenschaften e.V., 80539 München (DE)
(72) Erfinder: Geßner, Thomas, 69120 Heidelberg (DE); Reichelt, Helmut, 67435 Neustadt (DE); Müllen, Klaus, 50939 Köln (DE); Liu, Zhihong, Qingdao 266101 (DE); Li, Chen, 55128 Mainz (DE)
(86) Internationale Anmeldenummer: PCT/EP2009/056157
(87) Internationale Veröffentlichungsnummer: WO 2009/141387

(56) Entgegenhaltungen:
- EP-A- 0 657 436
- WO-A-01/16109
- WO-A-02/068538
- WO-A-2004/026965
- WO-A-2005/089094
- WO-A-2006/111511
- WO-A-2007/054470
- DE-A- 4 338 784
- Z. LIU ET AL.: "Amino-substituted rylene dicarboximides and their quinoidal charge delocalization after deprotonation" CHEM. COMMUN., Nr. 40, 4. September 2008 (2008-09-04), Seiten 5028-5030, XP002542330
- S. FERRERE, B. A. GREGG: "New perylenes for dye sensitization of TiO2" NEW JOURNAL OF CHEMISTRY, Bd. 29, Nr. 9, 30. Juli 2002 (2002-07-30), Seiten 1155-1160, XP002542331
- R. T. HAYES ET AL.: "Ultrafast Photoswitched Charge Transmission through the Bridge Molecule in a Donor-Bridge-Acceptor System" J. AM. CHEM. SOC., Bd. 122, Nr. 23, 23. Mai 2000 (2000-05-23), Seiten 5563-5567, XP002542332
- Y NAGAO, T. MISONO: "Synthesis and reactions of perylenecarboxylic acid derivatives. VI. Sulfonation of 3,4-perylenedicarboximide" BULLETIN OF THE CHEMICAL SOCIETY OF JAPAN, Bd. 52, Nr. 6, 1979, Seiten 1723-1726, XP002542333
- N. G. PSCHIRER ET AL.: "Pentarylene- and hexarylenebis(dicarboximide)s: near-infrared-absorbing polyaromatic dyes" ANGEWANDTE CHEMIE, INTERNATIONAL EDITION, Bd. 45, Nr. 9, 2006, Seiten 1401-1404, XP002542334
- S. FERRERE, B. A. GREGG: "Large Increases in Photocurrents and Solar Conversion Efficiencies by UV Illumination of Dye Sensitized Solar Cells" J. PHYS. CHEM. B, Bd. 105, Nr. 32, 19. Juli 2001 (2001-07-19), Seiten 7602-7605, XP002542335
- S. BECKER ET AL.: "New Thermotropic Dyes Based on Amino-Substituted Perylenedicarboximides" CHEM. EUR. J., Bd. 6, Nr. 21, 2000, Seiten 3984-3990, XP002542336
- M. J. FULLER, M. R. WASILIEWSKI: "Photorefractivity in Nematic Liquid Crystals Using a Donor-Acceptor Dyad with a Low-Lying Excited Singlet State for Charge Generation" J. PHYS. CHEM. B, Bd. 105, Nr. 30, 28. Juni 2001 (2001-06-28), Seiten 7216-7219, XP002542337

## Beschreibung

Die vorliegende Erfindung betrifft an der peri-Position Amino-substituierte Rylendicarboximide und deren Verwendung.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils konkret angegebenen Kombination, sondern auch in anderen Kombinationen verwendbar sind, ohne den Rahmen der Erfindung zu verlassen. Bevorzugt beziehungsweise ganz bevorzugt sind insbesondere auch diejenigen Ausführungsformen der vorliegenden Erfindung, in denen alle Merkmale des erfindungsgemäßen Gegenstandes die bevorzugten beziehungsweise ganz bevorzugten Bedeutungen haben.

Perylenimidverbindungen und ihre Homologen sind allgemein bekannt (L. Schmidt-Mende, et al., Science, 2001, 293, 1119; M.A. Angadi, et al., Mat.Sci. Eng., B, 1999, B63, 191).

In der EP 0 596 292 A1 werden beispielsweise Quaterrylencarbonsäurediimide, Verfahren zu ihrer Herstellung und ihre Verwendung als Fluoreszenzfarbstoffe beschrieben.

Perylen- und Terrylen-bis(dicarboximiide) absorbieren bekanntermaßen elektomagnetische Strahlung hauptsächlich im sichtbaren Bereich (F. Holtrup, et al., Chem.Eur.J., 1997, 3, 219-225; F. Nolde et al., Chem.Eur.J., 2005, 11, 3959), während die höheren Rylen-Homologe wie Quaterrylen-, Pentarylen- und Hexarylen-bis(dicarboximide) im Nahen Infra-Rot (NIR) absorbieren (H. Quante et al., Angew.Chem.Int.Ed., 1995, 34, 1323, N.G. Pschirer et al., Angew.Chem. 2006, 118, 1429).

WO 02/066438 A1 beschreibt Rylenderivate, Verfahren zur Herstellung der Rylenderivate und ihre Verwendung, beispielsweise zur Einfärbung von hochmolekularen organischen und anorganischen Materialien, als Dispergierhilfsmittel, als Pigmentadditive für organische Pigmente und Zwischenprodukte für die Herstellung von Fluoreszenzfarbmitteln und Pigmentadditiven. Weiterhin wird die Verwendung dieser Verbindungen als farbgebende Komponente in der dekorativen Kosmetik, zur Herstellung farbiger oder im nahinfraroten Bereich des elektromagnetischen Spektrums absorbierender und/oder emittierender wäßriger Polymerisatdispersionen, als Photoleiter in der Elektrophotographie, als Emitter in Elektro- und Chemilumineszenzanwendungen, als Aktivkomponenten in der Fluoreszenzkonversion und als Laserfarbstoffe beschrieben.

WO 04/029028 A1 beschreibt 9-Cyano substituierte Perylen-3,4-dicarbonsäuremonoimide, deren Herstellung und Verwendung.

Trotz der bereits beschriebenen Rylenderivate sowie deren Verwendung im Zusammenhang mit deren Absorptionsvermögen im NIR Spektralbereich besteht ein Bedarf an weiteren, insbesondere speziell substituierten Derivaten die im NIR absorbieren, die insbesondere einfach herzustellen und chemisch stabil sind.

Eine Aufgabe der vorliegenden Erfindung war es somit, die Bereitstellung solcher Rylenderivate, die insbesondere Vorzüge bei der Synthese und gute Eigenschaften aufgrund ihrer Stabilität und ihres Absorptionsvermögens aufweisen.

Diese Aufgabe wurde gelöst durch an der peri-Position Amino-substituierte Rylendicarboximide der Formel (I) oder Mischungen davon: 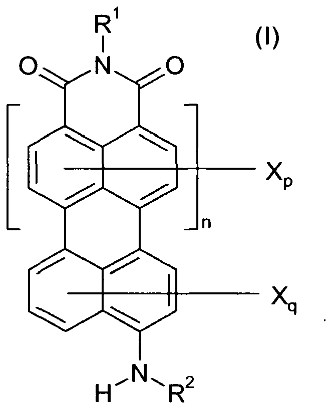 wobei
- R¹: H, C₁-C₂₀-Alkyl, C₃-C₁₅-Cycloalkyl, Aryl oder Hetaryl,
- R²: H, C₁-C₂₀-Alkyl, C₃-C₁₅-Cycloalkyl, Aryl oder Hetaryl,
- X: unabhängig voneinander, gleich oder verschieden, Halogen, C₁-C₂₀-Alkoxy,
- n: 1, 2, 3, 4, oder 5
- p, q: unabhängig voneinander, gleich oder verschieden 0, 1, 2, 3 oder 4
und wobei die Substituenten R¹ oder R² jeweils an beliebiger Position durch ein oder mehrere, gegebenenfalls durch Wasserstoff abgesättigte, Heteroatome unterbrochen sein können, wobei die Anzahl dieser Heteroatome nicht mehr als 10, bevorzugt nicht mehr als 8, ganz besonders bevorzugt nicht mehr als 6 und insbesondere nicht mehr als 4 beträgt, und/oder jeweils an beliebiger Position, allerdings nicht mehr als fünfmal, bevorzugt nicht mehr als viermal und besonders bevorzugt nicht mehr als dreimal, durch NR⁵R⁶, CONR⁵R⁶, COOM, COOR⁵, SO₃M, SO₃R⁵,
wobei,
- R⁵, R⁶: unabhängig voneinander, gleich oder verschieden, H, C₁-C₈-Alkyl, Aryl,
- M: H, Alkalimetall, NR⁷₄,
- R⁷: unabhängig voneinander H, C₁-C₈-Alkyl,

CN, NO₂, C₁-C₂₀-Alkyl, C₁-C₂₀-Alkoxy, Aryl, Aryloxy, Heterocyclen, Heteroatome oder Halogen substituiert sein können, wobei diese ebenfalls maximal zweimal, bevorzugt maximal einmal mit den genannten Gruppen substituiert sein können.

Es wurde gefunden, dass die oben beschriebenen Verbindungen der allgemeinen Formel (I), nach Deprotonierung am Amin-Stickstoff in peri-Position überraschend intensive Absorptionsbanden im NIR Spektralbereich aufweisen. Weiterhin zeigen diese Verbindungen eine hohe Stabilität, insbesondere im basischen Milieu.

Ausdrücke der Form Cₐ-C_{b} bezeichnen im Rahmen dieser Erfindung chemische Verbindungen oder Substituenten mit einer bestimmten Anzahl von Kohlenstoffatomen. Die Anzahl an Kohlenstoffatomen kann aus dem gesamten Bereich von a bis b, einschließlich a und b gewählt werden, a ist mindestens 1 und b immer größer als a. Eine weitere Spezifizierung der chemischen Verbindungen oder der Substituenten erfolgt durch Ausdrücke der Form Cₐ-C_{b}-V. V steht hierbei für eine chemische Verbindungsklasse oder Substituentenklasse, beispielsweise für Alkylverbindungen oder Alkylsubstituenten.

Als Infrarotstrahlung (kurz IR-Strahlung) werden im Rahmen der vorliegenden Erfindung elektromagnetische Wellen im Spektralbereich zwischen sichtbarem Licht und den längerwelligen Mikrowellen bezeichnet. Dies entspricht einem Wellenlängenbereich von etwa 760 nm bis 1 mm. Bei kurzwelliger IR-Strahlung (ab 760 nm) spricht man oft von nahem Infrarot (near infrared, NIR), bei Wellenlängen von ca. 5-25 Mikrometer von mittlerem Infrarot (mid infrared, MIR). Extrem langwellige IR-Strahlung (25 µm-1 mm) bezeichnet man als fernes Infrarot (far infrared, FIR). Infrarotstrahlung ist ein Teil der Wärmestrahlung.

Als sichtbares Licht werden im Rahmen der vorliegenden Erfindung elektromagnetische Wellen im Spektralbereich von etwa 380 nm bis 760 nm bezeichnet.

Substanzen die elektromagnetische Strahlung im Wellenlängenbereich der IR-Strahlung absorbieren werden im Rahmen der vorliegenden Erfindung auch als IR-Absorber bezeichnet. Bevorzugt weisen IR-Absorber eine Absorption im Wellenlängenbereich von 760 bis 2000 nm, ganz bevorzugt von 780 bis 1500 nm und einen Extinktionskoeffizienten für IR-Strahlung von mindestens 100 l/(cm * mol) auf. Bevorzugt liegt der Extinktionskoeffizienten für IR-Strahlung über 1000 l/(cm * mol) und ganz bevorzugt über 10⁴ l/(cm * mol).

Substanzen die sichtbares Licht absorbieren werden im Rahmen der vorliegenden Erfindung auch als farbig bezeichnet. Bevorzugt weisen farbige Substanzen einen Extinktionskoeffizienten für sichtbares Licht von mindestens 100 l/(cm * mol) auf. Bevorzugt liegt der Extinktionskoeffizienten für sichtbares Licht über 1000 l/(cm * mol) und ganz bevorzugt über 10⁴ l/(cm * mol).

Halogen steht für Fluor, Chlor, Brom, oder Iod, vorzugsweise für Fluor, Chlor oder Brom, besonders bevorzugt für Fluor oder Chlor.

Alkalimetalle sind Li, Na oder K. Insbesondere können die Alkalimetalle (M) in der chemischen Gruppe -SO₃M oder-COOM als einwertige positiv geladene Ionen auftreten.

Im Einzelnen haben die für die verschiedenen oben angegebenen Sammelbegriffe folgende Bedeutung:
C₁-C₂₀-Alkyl: geradkettige oder verzweigte Kohlenwasserstoffreste mit bis zu 20 Kohlenstoffatomen, beispielsweise C₁-C₁₀-Alkyl oder C₁₁-C₂₀-Alkyl, bevorzugt C₁-C₁₀-Alkyl beispielsweise C₁-C₈-Alkyl, C₁-C₃-Alkyl, wie Methyl, Ethyl, Propyl, Isopropyl, oder C₄-C₆-Alkyl, n-Butyl, sec-Butyl, tert.-Butyl, 1,1-Dimethylethyl, Pentyl, 2-Methylbutyl, 1,1-Dimethylpropyl, 1,2-Dimethylpropyl, 2,2-Dimethylpropyl, 1-Ethylpropyl, Hexyl, 2-Methylpentyl, 3-Methyl-pentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 2-Ethylbutyl, 1,1,2-Trimethylpropyl, 1,2,2-Tri-methylpropyl, 1-Ethyl-1-methylpropyl, 1-Ethyl-2-methylpropyl, oder C₇-C₁₀-Alkyl, wie Heptyl, Octyl, 2-Ethyl-hexyl, 2,4,4-Trimethylpentyl, 1,1,3,3-Tetramethylbutyl, Nonyl oder Decyl sowie deren Isomere.

C₁-C₂₀-Alkoxy bedeutet eine geradkettige oder verzweigte Alkylgruppe mit 1 bis 20 Kohlenstoffatomen (wie vorstehend genannt), welche über ein Sauerstoffatom (-O-) angebunden sind, beispielsweise C₁-C₁₀-Alkoxy oder C₁₁-C₂₀-Alkoxy, bevorzugt C₁-C₁₀-Alkyloxy, insbesondere bevorzugt C₁-C₃-Alkoxy, wie beispielweise Methoxy, Ethoxy, Propoxy.

C₃-C₁₅-Cycloalkyl: monocyclische, gesättigte Kohlenwasserstoffgruppen mit 3 bis zu 15 Kohlenstoffringgliedern, bevorzugt C₃-C₈-Cycloalkyl wie Cyclopropyl, Cyclobutyl, Cyclopentyl, Cyclohexyl, Cycloheptyl oder Cyclooctyl sowie ein gesättigtes oder ungesättigtes cyclisches System wie z. B. Norbornyl oder Norbenyl.

Aryl: ein ein- bis dreikerniges aromatisches Ringsystem enthaltend 6 bis 14 Kohlenstoffringglieder, z. B. Phenyl, Naphthyl oder Anthracenyl, bevorzugt ein ein- bis zweikerniges, besonders bevorzugt ein einkerniges aromatisches Ringsystem.

Aryloxy: ist ein ein- bis dreikerniges aromatisches Ringsystem (wie vorstehend genannt), welches über ein Sauerstoffatom (-O-) angebunden ist, bevorzugt ein ein- bis zweikerniges, besonders bevorzugt ein einkerniges aromatisches Ringsystem.

Heterocyclen (Heterocyclische Substituenten): fünf- bis zwölfgliedrige, bevorzugt fünfbis neungliedrige, besonders bevorzugt fünf- bis sechsgliedrige, Sauerstoff-, Stickstoff- und/oder Schwefelatome, gegebenenfalls mehrere Ringe aufweisende Ringsysteme wie Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl. Die Heterocyclen können in beliebiger Weise chemisch an die Verbindungen der allgemeinen Formel (I) angebunden sein, beispielsweise über eine Bindung zu einem Kohlenstoffatom des Heterocyclus oder eine Bindung zu einem der Heteroatome. Weiterhin, insbesondere, fünf- oder sechsgliedrige gesättigte stickstoffhaltige Ringsysteme, die über ein Ringstickstoffatom angebunden sind und die noch ein oder zwei weitere Stickstoffatome oder ein weiteres Sauerstoff- oder Schwefelatom enthalten können.

Hetaryl: Heterocyclische Substituenten, die sich formal von Aryl-Gruppen ableiten indem eine oder mehrere Methin- (-C=) und/oder Vinylengruppe (-CH=CH-) durch Tri- oder divalente Heteroatome ersetzt werden. Bevorzugt sind als Heteroatome Sauerstoff, Stickstoff und/oder Schwefel. Besonders bevorzugt Stickstoff und/oder Sauerstoff.

COOR¹: steht für Carbonsäuren (R¹ = H) oder Carbonsäureester (mit beispielsweise R¹= C₁-C₂₀-Alkyl oder Aryl).
COOM: steht für Salze von Carbonsäuren (beispielsweise einwertige Alkalimetallsalze).

SO₃R¹: steht für Sulfonsäuren (R¹ = H) oder Sulfonsäureester (mit beispielsweise R¹= C₁-C₂₀-Alkyl oder Aryl).
SO₃M: steht für Salze von Sulfonsäuren (beispielsweise einwertige Alkalimetallsalze).

CONR¹R²: steht für gegebenenfalls substituierte Carbonsäureamide. Beispielsweise sind in diesem Fall R¹ und R² gleich oder verschieden C₁-C₂₀-Alkyl oder Aryl.

Heteroatome sind Phosphor, Sauerstoff, Stickstoff oder Schwefel bevorzugt Sauerstoff, Stickstoff oder Schwefel.

Bevorzugt haben für die erfindungsgemäßen Verbindungen die Symbole in Formel (I) folgende Bedeutung:
- R¹: C₁-C₂₀-Alkyl, Aryl,
- R²: Aryl,
- X: Halogen, C₁-C₂₀-Alkoxy,
- n: 1, 2 oder 3
und alle anderen Symbole und Indizes haben die gleiche Bedeutung wie anfangs erwähnt.

Besonders bevorzugt haben für die erfindungsgemäßen Verbindungen die Symbole in Formel (I) folgende Bedeutung:
- R¹: C₁-C₂₀-Alkyl, Aryl,
- R²: Aryl,
- X: alle gleich Halogen oder C₁-C₂₀-Alkoxy,
- n: 1, 2 oder 3
- p, q: unabhängig voneinander, gleich oder verschieden 0, 1, 2
und alle anderen Symbole und Indizes haben die gleiche Bedeutung wie oben erwähnt. Ganz besonders bevorzugt sind p, q unabhängig voneinander, gleich oder verschieden 0 oder 2.

Die Verbindungen der allgemeinen Formel (I) können nach dem Fachmann geläufigen Methoden hergestellt werden, wie sie beispielsweise in T. Edvinsson, et al., J.Phys.Chem. C, 2007, 111, 15137 beschrieben sind.

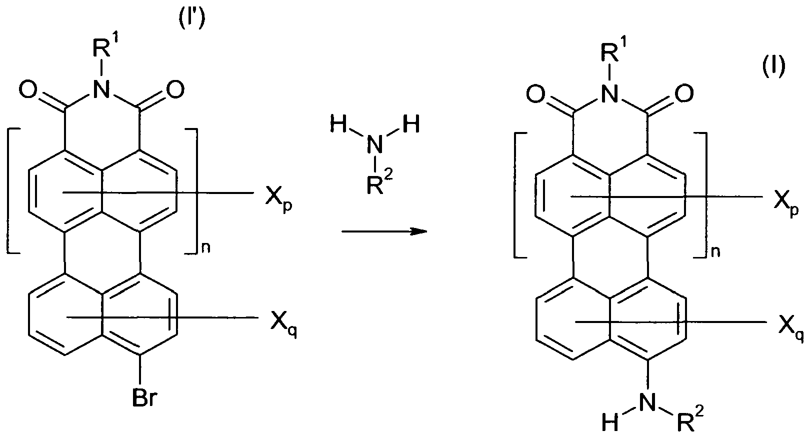

Verfahren zur Herstellung der Bromid-Ausgangsverbindungen (I') sind dem Fachmman ebenfalls beispielsweise bekannt aus: WO 2006/117383 A1; F. Holtrup, et al., Chem.Eur.J., 1997, 3, 219-225; F. Nolde et al., Chem.Eur.J., 2005, 11, 3959; H. Quante et al., Angew.Chem.Int.Ed., 1995, 34, 1323; N.G. Pschirer et al., Angew.Chem. 2006, 118, 1429 oder Y. Avlasevich, et al., J.Org.Chem., 2007, 72, 10243.

Die Verbindungen der allgemeinen Formel (I) werden erfindungsgemäß unter Verwendung von Basen am Amino-Substituenten in peri-Position deprotoniert.

Gegenstand der Erfindung sind daher auch die Verbindungen der allgemeinen Formel (II): 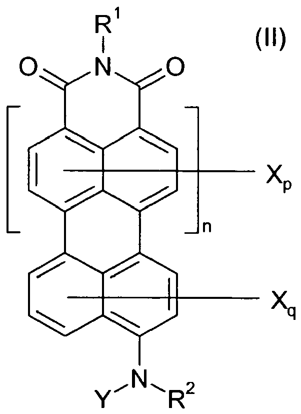 wobei
- Y: Alkalimetall, Erdalkalimetall, Ammonium, Alkylammonium
ist und die anderen Symbole, die gleiche Bedeutung wie eingangs für die Verbindungen der allgemeinen Formel (I) angegeben haben.

Bevorzugt haben für die erfindungsgemäßen Verbindungen die Symbole in Formel (II) folgende Bedeutung:
- R¹: C₁-C₂₀-Alkyl, Aryl,
- R²: Aryl,
- X: Halogen, C₁-C₂₀-Alkoxy,
- n: 1, 2 oder 3
und alle anderen Symbole und Indizes haben die gleiche Bedeutung wie oben erwähnt.

Besonders bevorzugt haben für die erfindungsgemäßen Verbindungen die Symbole in Formel (II) folgende Bedeutung:
- R¹: C₁-C₂₀-Alkyl, Aryl,
- R²: Aryl,
- X: alle gleich Halogen oder C₁-C₂₀-Alkoxy,
- n: 1, 2 oder 3
- p, q: unabhängig voneinander, gleich oder verschieden 0, 1, 2
und alle anderen Symbole und Indizes haben die gleiche Bedeutung wie oben erwähnt.

Ganz besonders bevorzugt sind p, q unabhängig voneinander, gleich oder verschieden 0 oder 2.

Die Deprotonierung der peri-Amingruppe ist im Allgemeinen reversibel und kann beispielsweise mit Säuren wieder rückgängig gemacht werden. Hierbei werden die Verbindungen der allgemeinen Formel (II) in die korrespondierenden Verbindungen der allgemeinen Formel (I) umgewandelt. Selbstverständlich können die erfindungsgemäßen Verbindungen einen oder mehrere Zyklen aus Deprotonierung und Protonierung durchlaufen. Ein Vorteil der erfindungsgemäßen Verbindungen (I) und (II) ist dabei, dass sie gegenüber einer Änderung des pH-Wertes oder der Konzentration von Protonen oder Hydroxidionen im Allgemeinen stabil sind und lediglich eine Deprotonierung oder Protonierung des peri-Amin-Substituenten erfolgt.

Im Allgemeinen wird die Deprotonierung in einem Lösungsmittel oder Lösungsmittelgemisch durchgeführt. Beispielsweise eignen sich als Lösungsmittel polare Lösungsmittel oder deren Mischungen, beispielsweise Wasser, Tetrahydrofuran, Ethanol, 2-Propanol, Aceton, Avetonitril, Dimethylformamid, N-Methyl-pyrrolidon oder Methylenchlorid. Bevorzugte Lösungsmittel sind Acton, Tetrahydrofuran, N-Methyl-pyrrolidon oder Methylenchlorid.

Als Basen lassen sich beispielsweise Alkalihydroxide, wie NaOH, KOH, Alkali-t-butylalkoholate, wie t-BuONa, t-BuOK, Kalium-bis-(trimethylsilyl)-amid, Ammoniak oder Alkylammoniumverbindungen einsetzen. Bevorzugt sind t-BuONa oder t-BuOK.

Besonders bevorzugt wird die Deporotonierung in Aceton mit t-BuONa oder t-BuOK durchgeführt.

Die Verbindungen der allgemeinen Formel (II) weisen gegenüber den protonierten Verbindungen der allgemeinen Formel (I) ein im Allgemeinen stark bathochrom verschobenes Absorptionsspektrum auf. Die Absorption der Verbindungen der allgemeinen Formel (II) ist im NIR Spektralbereich gegenüber den protonierten Verbindungen der allgemeinen Formel (I) im Allgemeinen deutlich stärker.

Daher eigenen sich die Verbindungen der allgemeinen Formeln (I) und (II) beispielsweise als sichtbare oder unsichtbare Markierstoffe.

Bevorzugt werden die erfindungsgemäßen bei der Markierung von Papier, Mineralöl, Kunststoffen oder Metalloberflächen eingesetzt. Besonders bevorzugt werden die Verbindungen der allgemeinen Formel (I) oder (II) als Markierstoffe für Mineralöle oder Papier eingesetzt. Ganz bevorzugt ist die Verwendung zur Markierung von Papier, insbesondere als Sicherheitsmerkmal für Dokumente, Wertpapiere oder Geldscheine.

Ein Vorteil der Verwendung als Markierstoff für Mineralöle ist, dass Mineralöle häufig in dem spektralen Absorptionsbereich der Verbindungen der allgemeinen Formel (II), beispielsweise im NIR, selbst keine Absorption aufweisen und ein ausgezeichnetes Signal/Rauschverhältnis für den Nachweis auch geringster Mengen des Markierstoffes, nach erfolgter Deprotonierung, erreicht wird.

Häufig absorbieren die Verbindungen der allgemeinen Formeln (I) selbst sichtbares Licht und können daher auch zur Anfärbung von Materialien, beispielsweise hochmolekularen organischen und anorganischen Materialien, insbesondere von Papier, Kunststoffen, Lacken oder Druckfarben eingesetzt werden. Diese Anfärbung kann durch Deprotonierung zu Verbindungen der allgemeinen Formel (II) reduziert oder entfernt werden, unter der Bedingung, dass das Absorptionsspektrum der deprotonierten Verbindungen eine bathochrome Verschiebung in den IR Bereich erfahren hat.

Ein weiterer Gegenstand der Erfindung ist ein Verfahren zur Markierung von Materialien, wobei die Materialien mit Verbindungen der allgemeinen Formeln (I) oder (II) in Kontakt gebracht werden. Der Nachweis der Markierung erfolgt in der Regel visuell (mit bloßem Auge) oder mit Hilfe eines (Absorptions)spektrometers.

Die erfindungsgemäße Markierung von Papier erfolgt beispielsweise durch das Aufbringen von Verbindungen der allgemeinen Formeln (I) und/oder (II) auf die Oberfläche des zu markierenden Papiers, beispielsweise durch Besprühen, Tränken oder Auftropfen mit Lösungen enthaltend die Verbindungen der Formeln (I) oder (II). Insbesondere lassen sich die Verbindungen der allgemeinen Formeln (I) oder (II) in einer Mischung mit einer Druckfarbe auf das Papier auftragen. Die Menge an Verbindungen der allgemeinen Formeln (I) oder (II) kann je nach Anwendung in einem breiten Bereich variieren. Bevorzugt werden weniger als 10 Gew.-% Verbindungen der allgemeinen Formeln (I) oder (II) bezogen auf die Menge an Druckfarbe eingesetzt, ganz bevorzugt weniger als 5 Gew.-%. Der Nachweis der Markierung kann beispielsweise durch einen Farbwechsel visuell oder mit Hilfe eines (Absorptions)spektrometers vorgenommen werden.

Die erfindungsgemäße Markierung von Mineralöl erfolgt durch Zugabe von Verbindungen der allgemeinen Formeln (I) oder (II) zum zu markierenden Mineralöl. Die Menge an Verbindungen der allgemeinen Formeln (I) oder (II) kann je nach Anwendung in einem breiten Bereich variieren. Bevorzugt werden weniger als 5 ppm Verbindungen der allgemeinen Formeln (I) oder (II) bezogen auf die Menge an Mineralöl eingesetzt, ganz bevorzugt weniger als 1 ppm. Der Nachweis der Markierung kann beispielsweise, optional nach einer Deprotonierung unter Einsatz von Basen, mit Hilfe eines (Absorptions)spektrometers vorgenommen werden.

Weiterhin lassen sich die erfindungsgemäßen Verbindungen der allgemeinen Formeln (I) oder (II) beim Laserschweißen einsetzen.

Ein weiterer Gegenstand der Erfindung ist daher ein Verfahren zum Laserschweißen von Materialien, wobei die Materialien zunächst mit Verbindungen der allgemeinen Formeln (I) oder (II) in Kontakt gebracht werden. Zum Verschweißen wird in einer bevorzugten Ausführungsform im Material durch Deprotonierung eine Zunahme in der IR-Absorption bewirkt, d.h. die erfindungsgemäßen Verbindungen der allgemeinen Formel (II) werden, wie unten beschrieben, als IR-Absorber eingesetzt.

Das Schweißen von Materialien, insbesondere von Kunststoffen erfolgt durch Absorption von Laserenergie im oder auf dem Kunststoffmaterial durch die zugesetzten lasersensitiven IR-Absorber, die durch Absorption der Laserenergie zu einer lokalen Erhitzung des Materials führen. Beim Laserscheißen von beispielsweise zwei Materialien wird im Fügebereich der zu verschweißenden Materialien durch Absorption der Laserenergie eine starke Erwärmung erzeugt, so dass die Materialien aufschmelzen und beide Materialien miteinander verschmelzen. Häufig ist es ausreichend, wenn nur eines der Materialien lasersensitive IR-Absorber im Material oder als Schicht an der Oberfläche enthält. Die Laserschweißbarkeit ist Abhängig von der Natur der Materialien, insbesondere Kunststoffe sowie von der Wellenlänge und der Strahlungsleistung des eingesetzten Lasers. Beispielsweise kommen für das erfindungsgemäße Verfahren zum Laserschweißen CO₂-, Excimer-, oder ND:YAG-Laser in Frage.

In der Regel beträgt der Gehalt an Verbindungen der allgemeinen Formeln (I) oder (II) insgesamt zwischen von 0,0001 bis 1 Gew.-% bezogen auf das zu verschweißende Material. Bevorzugt beträgt der Gehalt von 0,001 bis 0,1 Gew.-%. Insbesondere ergibt sich in diesem Bereich von 0,001 bis 0,1 Gew.-% eine ausreichende Verschweißbarkeit von Kunststoffen.

Die Verbindungen der allgemeinen Formeln (I) oder (II) können mit Hilfe von dem Fachmann bekannten Verfahren, wie beispielsweise durch Extrusion, in praktisch alle Kunststoffe eingearbeitet werden, insbesondere um diesen Laserschweißbarkeit zu verleihen. Typisch sind Kunststoffmaterialien bei denen die Kunststoffmatrix auf Poly(meth)acrylat, Polyamid, Polyurethan, Polyolefinen, Styrolpolymeren und Styrolcopolymeren, Polycarbonat, Silikonen, Polyimiden, Polysulfon, Polyethersulfon, Polyketone, Polyetherketone, PEEK, Polyphenylensulfid, Polyester (wie PET, PEN, PBT), Polyethylenoxid, Polyurethan, Polyolefinen, Cycloolefincopolymeren oder fluorhaltigen Polymeren (wie PVDF, EFEP, PTFE) basiert. Ebenfalls ist eine Einarbeitung in Blends möglich, die als Komponenten oben genannte Kunststoffe beinhalten, oder in von diesen Klassen abgeleitete Polymere, die durch nachträgliche Reaktionen verändert wurden. Diese Materialien sind in großer Vielfalt bekannt und kommerziell erhältlich.

Ein weiterer Gegenstand der Erfindung ist die Verwendung von Verbindungen der allgemeinen Formel (I) zum Nachweis von Basen. Durch die zu detektierenden Basen erfolgt eine Deprotonierung zu den korrespondierenden Verbindungen der allgemeinen Formel (II) und der Nachweis erfolgt beispielsweise durch eine visuell wahrnehmbare Farbänderung oder durch die Messung der Änderungen im Absorptionsspektrum mit Hilfe eine Spektrometers.

Ein weiterer Gegenstand der Erfindung ist die Verwendung von Verbindungen der allgemeinen Formel (II) zum Nachweis von Protonensäuren. Durch die zu detektierenden Protonensäuren erfolgt eine Protonierung zu den korrespondierenden Verbindungen der allgemeinen Formel (I) und der Nachweis erfolgt beispielsweise durch eine visuell wahrnehmbare Farbänderung oder durch die Messung der Änderungen im Absorptionsspektrum mit Hilfe eine Spektrometers.

Ein weiterer Gegenstand der vorliegenden Erfindung ist die Verwendung von Verbindungen der allgemeinen Formel (I) oder (II) als Dispergierhilfsmittel, Pigmentadditiv für organische Pigmente und Zwischenprodukte für die Herstellung von Pigmentadditiven.

Ein weiterer Gegenstand der vorliegenden Erfindung ist die Verwendung von Verbindungen der allgemeinen Formel (I) oder (II) im Wärmemanagement oder Energiemanagement.

Ein weiterer Gegenstand der vorliegenden Erfindung ist die Verwendung von Verbindungen der allgemeinen Formel (I) oder (II) in der Photovoltaik.

Ein weiterer Gegenstand der vorliegenden Erfindung ist die Verwendung von Verbindungen der allgemeinen Formel (I) oder (II) in der optischen Datenspeicherung.

Häufig sind die erfindungsgemäßen Verbindungen der allgemeinen Formeln (I) und (II) auch unter stark basischen Bedingungen stabil, während die Imid-Gruppen von anderen Rylen-Farbstoffen unter diesen Bedingungen Ringöffnungsreaktionen durchlaufen. Die deprotonierten Verbindungen der allgemeinen Formeln (II) weisen hohe Extinktionen im NIR-Spektralbereich auf.

Die Erfindung wird durch die Beispiele näher erläutert ohne dass die Beispiele den Gegenstand der Erfindung einschränken.

### Beispiele:

¹H, ¹³C, H, H COSY und NOE NMR wurden auf Bruker DPX 250, DRX 500 und Avance 700 NMR Spektrometern aufgenommen. Infrarorspektren wurden mit einem Nicolet FT IR320 Spektrometer erhalten. FD Massenspektren wurden mit einem VGinstruments ZAB 2-SE-FPD Gerät erhalten. MALDI-TOF wurden auf einem Bruker MALDI-TOF Spektrometer aufgenommen. UV/Vis/NIR wurden in 1-cm Quarzküvetten mit einem Perkin-Elmer Lambda 900 Spektrophotometer aufgenommen.

### Herstellung von Verbindungen der allgemeinen Formel (I):

### Beispiel 1:

(A) 4-Aminobenonitril wird durch Pd-katalyse mit der entsprechenden peri-Brom Verbindung unter Buchwaldbedingungen umgesetzt (vgl. T. Edvinsson, et al., J.Phys.Chem. C, 2007, 111, 15137). Reagenzien: 4-Aminpbenzonitril, Pd₂(dba)₃, Tris-tert-buthylphosphine, t-BuONa, Toluol, Umsetzung bei 80°C, 12h. Ausbeute: 84% d.Th. (der Theorie).

### Umsetzung unter Buchwald Bedingungen:

Die peri-Brom Verbindung (0.18 mmol), 4-Aminobenzonitrile (0.36 mmol), Tris-(dibenzylideneaceton)-dipalladium(0) (17 mg, 0.018 mmol), Tris-tert-butylphosphine (18 mg, 0.09mmol), Natrium-tert-butoxide (67 mg, 0.69 mmol) and trockenes Toluol (10 ml) wurden bei 80°C unter Argonatmosphäre über Nacht gerührt. Nach dem Abkühlen wurde die Mischung im Vakuum abgedampft und Anschliessend durch Säulenchromatographi gereinigt (Silikagel mit Dichlormethan als Eluent).

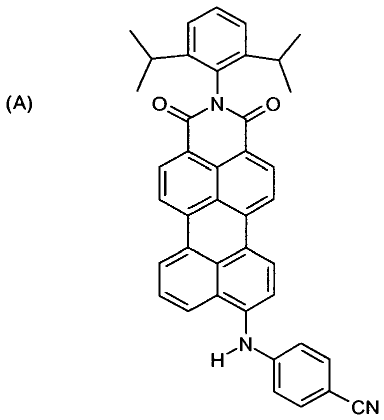

### N-(2, 6-Diisopropylphenyl)-9-(p-cyanphenyl-amino)-perylen-3,4-dicarboximid (A):

¹H NMR (700 MHz, Aceton-d₆, 50°C): δ = 8.78 (d, J=7.5 Hz, 1H), 8.72 (d, J=8.0 Hz, 1H), 8.70 (d, J=8.3 Hz, 1H), 8.63 (d, J=8.0 Hz, 1H), 8.60 (d, J=8.0 Hz, 1H), 8.58 (d, J=8.0 Hz, 1H), 8.49 (s, 1 N-H), 8.35 (d, J=8.4 Hz, 1H), 7.76-7.74 (m, 2H), 7.66 (d, J=8.5 Hz, 2H), 7.44 (t, J=7.9 Hz, 1H), 7.36-7.33 (m, 4H), 2.83 (sep, J=6.8 Hz, 2H), 1.16 ppm (d, J=6.9 Hz, 12H); H,H COSY NMR (700 MHz, Aceton-d₆, 50°C): Kopplung von δ = (8.78, 8.35, 7.76 ), (8.70, 7.76), (8.72, 8.60), (8.63, 8.58), (7.66, 7.36), (7.44,7.36), (2.83, 1.16); NOE NMR (700 MHz, Aceton-d₆, 50°C): Kopplung von δ = (8.78, 8.72), (8.70, 8.63), (8.49, 7.36), (8.49, 8.35), (7.76, 7.36); ¹³C-NMR (62.5 MHz, CD₂Cl₂ 25°C): δ = 164.6, 164.4, 148.0, 146.5, 139.8, 137.8, 134.3, 132.3, 132.12, 132.0, 130.2, 129.7, 128.6, 127.4, 125.7, 125.2, 125.0, 124.7, 124.4, 121.5, 120.8, 120.5, 120.0, 119.7, 118.7, 117.2, 103.6, 29.5, 24.1 ppm. IR: v = 3328, 3064, 2962, 2929, 2867, 2217, 1689, 1644, 1563, 1504, 1351, 1288, 1172, 1058, 971, 910, 804, 750 cm⁻¹; UV-Vis (Aceton) λₘₐₓ, nm (ε): 550 (34800); MS (FD): m/z, 598.0 (100%), M⁺;
Elementaranalyse: Gefunden: C, 81.75; H, 5.42; N, 6.98 %. Berechnet für C₄₁H₃₁N₃O₂: C, 82.39; H, 5.23; N, 7.03 %.

Die zu (A) zugehörige deprotonierte Verbindung (A') wurde in quantitativer Ausbeute durch die Umsetzung mit einem Äquivalent NaOH in Aceton bei Raumtemperatur (20°C) erhalten:

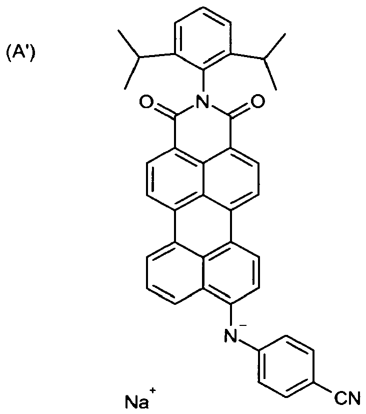

(A'): ¹H-NMR (700 MHz, Aceton-d₆, 25°C): δ = 8.81 (d, J=7.7 Hz, 1H), 8.65 (d, J=7.5 Hz, 1H), 8.29 (d, J=8.3 Hz, 1H), 8.18 (d, J=8.4 Hz, 1H), 8.09 (d, J=8.8 Hz, 1H), 8.06 (d, J=9.7 Hz, 1H), 7.63 (d, J=8.6 Hz, 1H), 7.57 (d, J=8.4 Hz, 2H), 7.45 (t, J=7.7 Hz, 1H), 7.32 (t, J=7.6 Hz, 1H), 7.25 (d, J=7.8 Hz, 2H), 7.10 (d, J=8.4 Hz, 2H), 6.60 (d, J=9.4 Hz, 1H), 2.83 (sep, J=6.8 Hz, 2H), 1.16 ppm (d, J=6.9Hz, 12H); H,H COSY NMR (700 MHz, Aceton-d₆, 25°C): Kopplung von δ = (8.81, 8.65, 7.45), (8.29, 8.18), (8.09, 7.63), (8.06, 6.60), (7.57, 7.10), (7.32, 7.25), (2.83, 1.16); NOE NMR (700 MHz, Aceton-d₆, 25°C): Kopplung von δ = (8.65, 8.18), (8.06, 7.63), (7.10, 6.60).

(A') ließ sich durch den Einsatz von einem Äquivalent HCl in Aceton bei Raumtemperatur quantitativ wieder in (A) umwandeln.

In analoger Weise wurden die Verbindungen (B) (Ausbeute 55%) und (C) (Ausbeute 53%) bzw. die korrespondierenden deprotonierten Verbindungen (B') und (C') hergestellt.

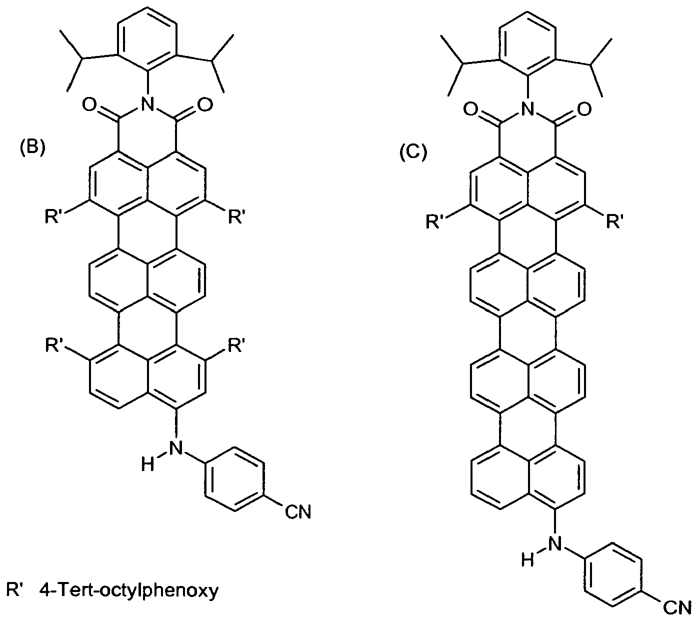

### N-(2,6-Diisopropylphenyl)-1,6,9,14-tetrakis[4-(1,1,3,3-tetramethylbutyl)phenoxy]-11-(p-cyanophenyl-amino)-terrylen-3,4-dicarboximid (B):

¹H NMR (250 MHz, Aceton-d₆, 25°C): δ = 9.50-9.45 (dd, 2H), 9.22-9.13 (dd, 2H) 8.35 (d, J=8.9Hz, 1H), 8.17 (d, J=8.9Hz, 1H), 7.68-7.52 (m, 10H), 7.46 (d, J=7.2Hz, 2H), 7.38-7.28 (m, 7H), 7.39-7.11 (m, 6H), 2.92 (sep, J=6.8Hz, 2H), 1.95-1.89 (q, 8H ), 1.55-1.49 (q, 24H), 1.23-1.19 (q, 12H), 1.01 (d, J=6.9Hz, 12H), 0.86-0.83 ppm (q, 24H); IR: v = 3326, 3054, 2952, 2923, 2865, 2221, 1698, 1594, 1502, 1365, 1303, 1211, 1170, 1052, 1014, 958, 829, 727 cm⁻¹; UV-Vis (Aceton) λₘₐₓ, nm (ε): 668 (41900); MS (FD): m/z, 1539.3 (100%), M⁺.

### N-(2,6-Diisopropylphenyl)-1,6-bis[4-(1,1,3,3-tetramethylbutyl)phenoxy]-13-(p-cyanophenyl-amino)-quaterrylen-3,4-dicarboximid (C):

IR: v =3359, 3054, 2954, 2925, 2865, 2211, 1693, 1577, 1500, 1384, 1317, 1265, 1209, 1170, 1064, 1014, 806, 744 cm⁻¹; UV-Vis (Aceton) λₘₐₓ, nm (ε): 751 (51200); MS (FD): m/z, 1255.3 (100%), M⁺.

Die Verbindungen (D) (Ausbeute 77%) bzw. (D') wurden in analoger Weise hergestellt, statt 4-Aminobenzonitril kam 4-Octylanilin zum Einsatz.

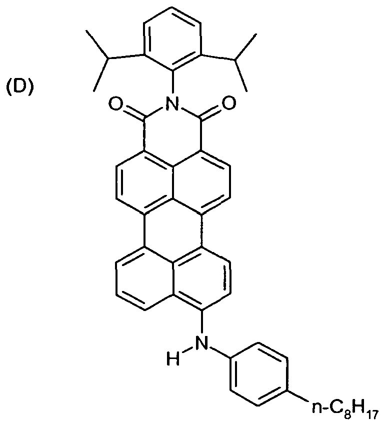

### N-(2, 6-Diisopropylphenyl)-9-(p-octylphenyl-amino)-perylen-3,4-dicarboximid (D):

¹H NMR (700 MHz, Aceton-d₆, 50°C): δ = 8.75 (d, J=7.3 Hz, 1H), 8.63 (d, J=8.1 Hz, 1H), 8.55 (d, J=7.9 Hz, 1H), 8.54 (d, J=8.6 Hz, 1H), 8.50 (d, J=8.1 Hz, 1H), 8.48 (d, J=8.5 Hz, 1H), 8.42 (d, J=8.2 Hz, 1H), 8.12 (s, 1 N-H), 7.73 (t, J=8.2 Hz, 1H), 7.45 (t, J=7.9 Hz, 1H), 7.38 (d, J=8.5 Hz, 1H), 7.34 (d, J=7.9 Hz, 2H), 7.34 (d, J=8.4 Hz, 2H), 7.29 (d, J=8.4 Hz, 2H), 2.87-2.70 (sep, J=6.8 Hz, 2H), 2.67-2.61 (t, J=7.7 Hz, 2H ), 1.68-1.62 (t, J=7.4 Hz, 2H), 1.34-1.30 (m, 10H), 1.18 (d, J=6.9 Hz, 12H), 0.92-0.87 ppm (t, J=7.1 Hz, 3H); H,H COSY NMR (700 MHz, Aceton-d₆, 50°C): Kopplung von δ = (7.73, 8.75, 8.48), (7.38, 8.54), (7.29, 7.34), (7.45, 7.34), (8.50, 8.42), (8.55, 8.63), (2.87, 1.18), (2.67, 1.68), (1.62, 1.34), (1.30, 0.92); ¹³C-NMR (62.5 MHz, CD₂Cl₂ 25°C): δ = 164.71, 164.55, 146.52, 144.34, 139.16, 138.96, 138.71, 138.17, 132.27, 131.88, 131.07, 129.97, 129.76, 129.16, 129.56, 129.16, 126.68, 126.47, 126.47, 125.11, 125.02, 124.35, 123.61, 121.84, 120.92, 120.83, 119.88, 118.69, 118.48, 111.08, 35.74, 32.30, 32.06, 29.89, 29.73, 29.69, 29.45, 24.14, 24.10, 23.08, 14.27 ppm. IR: v = 3372, 2964, 2924, 2854, 1690, 1650, 1566, 1514, 1354, 1284, 804,750 cm⁻¹; UV-Vis (Aceton) λₘₐₓ, nm (ε): 596 (35233); MS (FD): m/z 683.5 (100%), M⁺; Elementaranalyse: Gefunden: C, 83.39; H, 7.06; N, 3.96 %. Berechnet für C₄₈H₄₈N₂O₂: C, 84.17; H, 7.06; N, 4.09 %.

(D'): ¹H NMR (250 MHz, Aceton-d₆, 25 °C): δ = 8.83 (d, J=7.6 Hz, 1H), 8.67 (d, J=8.5 Hz, 1H), 8.25 (d, J=8.5 Hz, 1H), 8.11 (d, J=8.7 Hz, 1H), 7.99 (d, J=9.0 Hz, 1H), 7.94 (d, J=10.1 Hz, 1H), 7.48 (d, J=9.0 Hz, 1H), 7.46 (t, J=7.7 Hz, 1H), 7.32 (t, J=7.9 Hz, 1H), 7.25 (d, J=7.9 Hz, 2H), 7.16 (d, J=8.4 Hz, 2H), 6.89 (d, J=8.4 Hz, 2H), 6.52 (d, J=10.0 Hz, 1H), 2.77-2.66 (sep, J=6.9 Hz, 4H), 2.63-2.56 (t, J=7.4 Hz, 2H ), 1.64-1.59 (t, J=7.4 Hz, 2H), 1.34-1.30 (m, 10H), 1.13-1.10 (d, J=6.9 Hz, 12H), 0.92-0.86 ppm (t, J=7.0 Hz, 3H); H,H COSY NMR (700 MHz, Aceton-d₆, 25°C): Kopplung von δ = (7.50, 8.83, 8.67), (6.87, 7.12), (6.52, 6.48), (7.35, 7.25), (7.99, 7.48), (8.11, 8.25), (2.77, 1.13), (2.63, 1.64), (1.64, 1.34), (1.30, 0.92).

### Beispiel 2:

Verbindung (D') war gegenüber einer Behandlung mit einem Überschuss an NaOH in 2-Propanol unter Rückflussbedingungen stabil.

### Beispiel 3:

λ_{max:} Wellenlänge des Absorptionsmaximums [nm]
ε: Extinktionskoeffizient [M⁻¹ cm⁻¹]

Absorptionsmaxima der Verbindungen (A)-(D) bzw. (A')-(D'):

| Verbindung | λₘₐₓ [nm], (ε) | Verbindung | λₘₐₓ [nm] , (ε) |
|---|---|---|---|
| A | 550 (34800) | A' | 826 (90400) |
| D | 596 (35200) | D' | 780 (70400) |
| B | 668 (41900) | B' | 1008 (132200) |
| C | 751 (51200) | C' | 1186 (65100) |

Die Verbindung D' war in einer wässrigen Acetonlösung für 3 Tage stabil und erlitt lediglich einen Intensitätsverlust von 5%.

Wiederholte Titrationen (fünf Zyklen) der Verbindung D in Aceton mit wässriger NaOH (0,1 molar) und HCL (0,1 molar) zeigten die Reversibilität der Protonierung und Deprotonierung.

## Patentansprüche

1. Verbindungen der allgemeinen Formel (II) 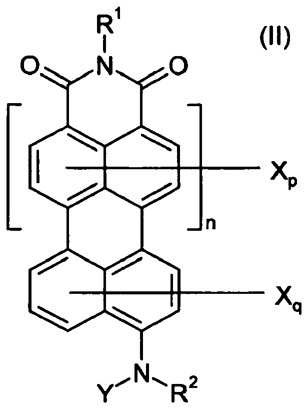 wobei
Y Alkalimetall, Erdalkalimetall, Ammonium, Alkylammonium
R¹ H, C₁-C₂₀-Alkyl, C₃-C₁₅-Cycloalkyl, Aryl oder Hetaryl,
R² H, C₁-C₂₀-Alkyl, C₃-C₁₅-Cycloalkyl, Aryl oder Hetaryl,
X unabhängig voneinander, gleich oder verschieden, Halogen, C₁-C₂₀-Alkoxy,
n 1, 2, 3, 4, oder 5
p, q unabhängig voneinander, gleich oder verschieden 0,1, 2, 3 oder 4
und wobei die Substituenten R¹ oder R² jeweils an beliebiger Position durch ein oder mehrere, gegebenenfalls durch Wasserstoff abgesättigte, Heteroatome unterbrochen sein können, wobei die Anzahl dieser Heteroatome nicht mehr als 10, bevorzugt nicht mehr als 8, ganz besonders bevorzugt nicht mehr als 6 und insbesondere nicht mehr als 4 beträgt, und/oder jeweils an beliebiger Position, allerdings nicht mehr als fünfmal, bevorzugt nicht mehr als viermal und besonders bevorzugt nicht mehr als dreimal, durch NR⁵R⁶, CONR⁵R⁶, COOM, COOR⁵, SO₃M, SO₃R⁵,
wobei,
R⁵, R⁶ unabhängig voneinander, gleich oder verschieden, H, C₁-C₈-Alkyl, Aryl,
M H, Alkalimetall, NR⁷₄,
R⁷ unabhängig voneinander H, C₁-C₈-Alkyl,
CN, NO₂, C₁-C₂₀-Alkyl, C₁-C₂₀-Alkoxy, Aryl, Aryloxy, Heterocyclen, Heteroatome oder Halogen substituiert sein können, wobei diese ebenfalls maximal zweimal, bevorzugt maximal einmal mit den genannten Gruppen substituiert sein können.

2. Verbindungen gemäß Anspruch 1, mit
R¹ C₁-C₂₀-Alkyl, Aryl,
R² Aryl,
X alle gleich Halogen oder C₁-C₂₀-Alkoxy,
n 1, 2 oder 3
wobei alle anderen Symbole und Indizes haben die gleiche Bedeutung wie in Anspruch 1 haben.

3. Verbindungen der allgemeinen Formel (I) 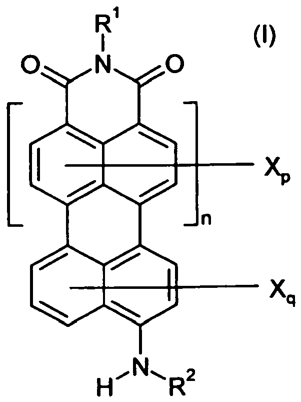 wobei
R¹ H, C₁-C₂₀-Alkyl, C₃-C₁₅-Cycloalkyl, Aryl oder Hetaryl,
R2 Aryl oder Hetaryl,
X unabhängig voneinander, gleich oder verschieden, Halogen, C₁-C₂₀-Alkoxy,
n 1, 2, 3, 4, oder 5
p, q unabhängig voneinander, gleich oder verschieden 0,1, 2, 3 oder 4
und wobei die Substituenten R¹ oder R² jeweils an beliebiger Position durch ein oder mehrere, gegebenenfalls durch Wasserstoff abgesättigte, Heteroatome unterbrochen sein können, wobei die Anzahl dieser Heteroatome nicht mehr als 10, bevorzugt nicht mehr als 8, ganz besonders bevorzugt nicht mehr als 6 und insbesondere nicht mehr als 4 beträgt, und/oder jeweils an beliebiger Position, allerdings nicht mehr als fünfmal, bevorzugt nicht mehr als viermal und besonders bevorzugt nicht mehr als dreimal, durch NR⁵R⁶, CONR⁵R⁶, COOM, COOR⁵, SO₃M, SO₃R⁵,
wobei,
R⁵, R⁶ unabhängig voneinander, gleich oder verschieden, H, C₁-C₈-Alkyl, Aryl,
M H, Alkalimetall, NR⁷₄,
R⁷ unabhängig voneinander H, C₁-C₈-Alkyl,
CN, NO₂, C₁-C₂₀-Alkyl, C₁-C₂₀-Alkoxy, Aryl, Aryloxy, Heterocyclen, Heteroatome oder Halogen substituiert sein können, wobei diese ebenfalls maximal zweimal, bevorzugt maximal einmal mit den genannten Gruppen substituiert sein können.

4. Verbindungen gemäß Anspruch 3, mit
R¹ C₁-C₂₀-Alkyl, Aryl,
R² Aryl,
X alle gleich Halogen oder C₁-C₂₀-Alkoxy,
n 1, 2 oder 3
wobei alle anderen Symbole und Indizes haben die gleiche Bedeutung wie in Anspruch 3 haben.

5. Verwendung von Verbindungen gemäß Ansprüchen 1 bis 4 als sichtbare oder unsichtbare Markierstoffe.

6. Verwendung nach Anspruch 5, wobei die Verbindungen gemäß Ansprüchen 1 bis 4 bei der Markierung von Papier, Mineralöl, Kunststoffen oder Metalloberflächen eingesetzt werden.

7. Verwendung von Verbindungen gemäß Ansprüchen 1 bis 4 zur Anfärbung von Materialien.

8. Verwendung nach Anspruch 7, wobei die Verbindungen gemäß Ansprüchen 1 bis 4 bei der Anfärbung von hochmolekularen organischen und anorganischen Materialien eingesetzt werden.

9. Verfahren zur Markierung von Materialien, **dadurch gekennzeichnet, dass** die Materialien mit Verbindungen gemäß den Ansprüchen 1 bis 4 in Kontakt gebracht werden.

10. Verwendung von Verbindungen gemäß Ansprüchen 1 bis 4 beim Laserschweißen von Materialien.

11. Verfahren zum Laserschweißen von Materialien, wobei die Materialien zunächst mit Verbindungen gemäß den Ansprüchen 1 bis 4, die als IR-Absorber dienen, in Kontakt gebracht werden, optional durch Deprotonierung eine Zunahme in der IR-Absorption erfolgt und das Material mit einem Laser bestrahlt wird, dessen emittierte Wellenlänge mit dem Absorptionsbereich der IR-Absorber überlappt.

12. Verwendung von Verbindungen gemäß Ansprüchen 1 bis 4 zum Nachweis von Basen, Säuren oder pH-Wert Änderungen.

13. Verwendung von Verbindungen gemäß Ansprüchen 1 bis 4 als Dispergierhilfsmittel, Pigmentadditiv für organische Pigmente und Zwischenprodukte für die Herstellung von Pigmentadditiven.

14. Verwendung von Verbindungen gemäß Ansprüchen 1 bis 4 im Wärmemanagement oder Energiemanagement.

15. Verwendung von Verbindungen gemäß Ansprüchen 1 bis 4 in der Photovoltaik.

16. Verwendung von Verbindungen gemäß Ansprüchen 1 bis 4 in der optischen Datenspeicherung.

## Claims

1. A compound of the general formula (II) 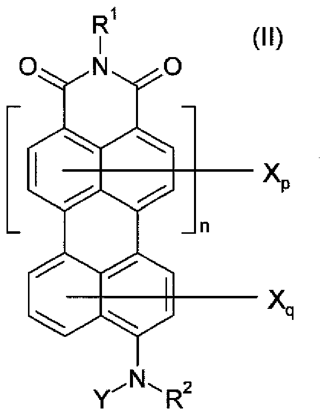 where
Y is alkali metal, alkaline earth metal, ammonium, alkylammonium,
R¹ is H, C₁-C₂₀-alkyl, C₃-C₁₅-cycloalkyl, aryl or hetaryl,
R² is H, C₁-C₂₀-alkyl, C₃-C₁₅-cycloalkyl, aryl or hetaryl,
X is the same or different and is independently halogen, C₁-C₂₀-alkoxy,
n is 1, 2, 3, 4 or 5
p, q are the same or different and are each independently 0, 1 , 2, 3 or 4,
and where the substituents R¹ or R² may each be interrupted at any position by one or more heteroatoms optionally satisfied by hydrogen, where the number of these heteroatoms is not more than 10, preferably not more than 8, even more preferably not more than 6 and especially not more than 4, and/or may each be substituted at any position, but not more than five times, preferably not more than four times and more preferably not more than three times, by NR⁵R⁶, CONR⁵R⁶, COOM, COOR⁵, SO₃M, SO₃R⁵,
where
R⁵, R⁶ are the same or different and are each independently H, C₁-C₈-alkyl, aryl,
M is H, alkali metal, NR⁷₄,
R⁷ is independently H, C₁-C₈-alkyl,
CN, NO₂, C₁-C₂₀-alkyl, C₁-C₂₀-alkoxy, aryl, aryloxy, heterocycles, heteroatoms or halogen, each of which may likewise be substituted not more than twice, preferably not more than once, by the groups specified.

2. A compound according to claim 1, where
R¹ is C₁-C₂₀-alkyl, aryl,
R2 is aryl,
X are all halogen or C₁-C₂₀-alkoxy,
n is 1, 2 or 3,
where all other symbols and indices are each as defined in claim 1.

3. A compound of the general formula (I) 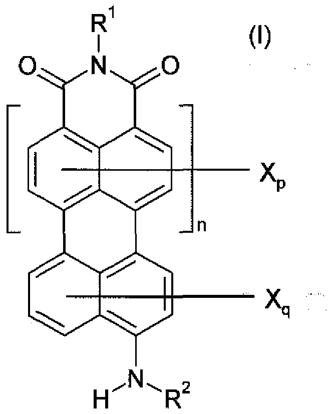 where
R¹ is H, C₁-C₂₀-alkyl, C₃-C₁₅-cycloalkyl, aryl or hetaryl,
R² is aryl or hetaryl,
X is the same or different and is independently halogen, C₁-C₂₀-alkoxy,
n is 1, 2, 3, 4 or 5
p, q are the same or different and are each independently 0,1, 2, 3 or 4,
and where the substituents R¹ or R² may each be interrupted at any position by one or more heteroatoms optionally satisfied by hydrogen, where the number of these heteroatoms is not more than 10, preferably not more than 8, even more preferably not more than 6 and especially not more than 4, and/or may each be substituted at any position, but not more than five times, preferably not more than four times and more preferably not more than three times, by NR⁵R⁶, CONR⁵R⁶, COOM, COOR⁵, SO₃M, SO₃R⁵,
where
R⁵, R⁶ are the same or different and are each independently H, C₁-C₈-alkyl, aryl,
M is H, alkali metal, NR⁷₄,
R⁷ is independently H, C₁-C₈-alkyl,
CN, NO₂, C₁-C₂₀-alkyl, C₁-C₂₀-alkoxy, aryl, aryloxy, heterocycles, heteroatoms or halogen, each of which may likewise be substituted not more than twice, preferably not more than once, by the groups specified.

4. A compound according to claim 3, where
R¹ is C₁-C₂₀-alkyl, aryl,
R² is aryl,
X are all halogen or C₁-C₂₀-alkoxy,
n is 1, 2 or 3,
where all other symbols and indices are each as defined in claim 3.

5. The use of compounds according to claims 1 to 4 as visible or invisible markers.

6. The use according to claim 5, wherein the compounds according to claims 1 to 4 are used in the marking of paper, mineral oil, plastics or metal surfaces.

7. The use of compounds according to claims 1 to 4 for staining materials.

8. The use according to claim 7, wherein the compounds according to claims 1 to 4 are used in the staining of high molecular weight organic and inorganic materials.

9. A process for marking materials, which comprises contacting the materials with compounds according to claims 1 to 4.

10. The use of compounds according to claims 1 to 4 in the laser welding of materials.

11. A process for laser welding materials, wherein the materials are first contacted with compounds according to claims 1 to 4 which serve as IR absorbers, the IR absorption is optionally increased by deprotonation, and the material is irradiated with a laser whose emitted wavelength overlaps with the absorption region of the IR absorbers.

12. The use of compounds according to claims 1 to 4 for detecting bases, acids or pH changes.

13. The use of compounds according to claims 1 to 4 as a dispersing assistant, pigment additive for organic pigments and intermediates for the production of pigment additives.

14. The use of compounds according to claims 1 to 4 in heat management or energy management.

15. The use of compounds according to claims 1 to 4 in photovoltaics.

16. The use of compounds according to claims 1 to 4 in optical data storage.

## Revendications

1. Composés de formule générale (II) 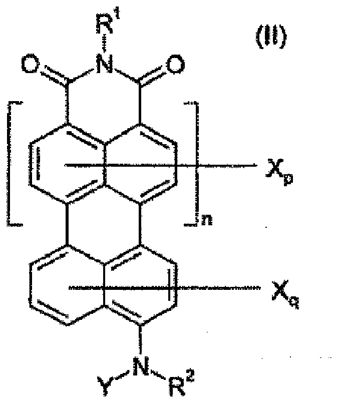 dans laquelle
Y représente un atome de métal alcalin, de métal alcalino-terreux, un ion ammonium, alkylammonium,
R¹ représente H, un groupe alkyle en C₁-C₂₀, cycloalkyle en C₃-C₁₅, aryle ou hétéroaryle,
R² représente H, un groupe alkyle en C₁-C₂₀, cycloalkyle en C₃-C₁₅, aryle ou hétéroaryle,
X le même ou différent, représente chaque fois indépendamment un atome d'halogène, un groupe alcoxy en C₁-C₂₀,
n vaut 1, 2, 3, 4 ou 5,
p, q identiques ou différents, représentent, indépendamment l'un de l'autre, 0, 1, 2, 3 ou 4
et dans laquelle les substituants R¹ ou R² pouvant être interrompus chacun en position quelconque par un ou plusieurs hétéroatomes éventuellement saturés par des atomes d'hydrogène, le nombre de ces hétéroatomes n'étant pas supérieur à 10, de préférence pas supérieur à 8, de façon tout particulièrement préférée pas supérieur à 6 et en particulier pas supérieur à 4, et/ou peuvent être substitués chacun en position quelconque, mais pas plus de cinq fois, de préférence pas plus de quatre fois et de façon particulièrement préférée pas plus de trois fois, par NR⁵R⁶, CONR⁵R⁶, COOM, COOR⁵, SO₃M, SO₃R⁵,
R⁵, R⁶ identiques ou différents, représentant, indépendamment l'un de l'autre, H, un groupe alkyle en C₁-C₈, aryle,
M représentant H, un métal alcalin, NR⁷₄,
R⁷ représentant chaque fois indépendamment H, un groupe alkyle en C₁-C₈,
CN, NO₂, alkyle en C₁-C₂₀, alcoxy en C₁-C₂₀, aryle, aryloxy, des hétérocycles, des hétéroatomes ou des atomes d'halogène, ceux-ci pouvant également être substitués au maximum deux fois, de préférence au maximum une fois, par les groupes nommés.

2. Composés selon la revendication 1, dans lesquels
R¹ représente un groupe alkyle en C₁-C₂₀, aryle,
R² représente un groupe aryle,
X représentent tous un atome d'halogène ou un groupe alcoxy en C₁-C₂₀,
n vaut 1, 2 ou 3,
tous les autres symboles et indices ayant la même signification que dans la revendication 1.

3. Composés de formule générale (I) 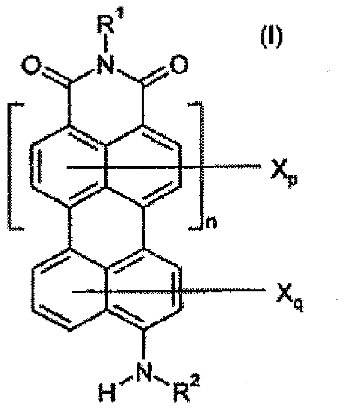 dans laquelle
R¹ représente H, un groupe alkyle en C₁-C₂₀, cycloalkyle en C₃-C₁₅, aryle ou hétéroaryle,
R² représente un groupe aryle ou hétéroaryle,
X le même ou différent, représente chaque fois indépendamment un atome d'halogène, un groupe alcoxy en C₁-C₂₀,
n vaut 1, 2, 3, 4 ou 5,
p, q identiques ou différents, représentent, indépendamment l'un de l'autre, 0, 1, 2, 3 ou 4
et les substituants R¹ ou R² peuvent être interrompus chacun en position quelconque par un ou plusieurs hétéroatomes éventuellement saturés par des atomes d'hydrogène, le nombre de ces hétéroatomes n'étant pas supérieur à 10, de préférence pas supérieur à 8, de façon tout particulièrement préférée pas supérieur à 6 et en particulier pas supérieur à 4, et/ou peuvent être substitués chacun en position quelconque, mais pas plus de cinq fois, de préférence pas plus de quatre fois et de façon particulièrement préférée pas plus de trois fois, par NR⁵R⁶, CONR⁵R⁶, COOM, COOR⁵, SO₃M, SO₃R⁵,
R⁵ R⁶ identiques ou différents, représentant, indépendamment l'un de l'autre, H, un groupe alkyle en C₁-C₈, aryle,
M représentant H, un métal alcalin, NR⁷₄,
R⁷ représentant chaque fois indépendamment H, un groupe alkyle en C₁-C₈,
CN, NO₂, allyle en C₁-C₂₀, alcoxy en C₁-C₂₀, aryle, aryloxy, des hétérocycles, des hétéroatomes ou des atomes d'halogène, ceux-ci pouvant également être substitués au maximum deux fois, de préférence au maximum une fois, par les groupes nommés.

4. Composés selon la revendication 3, dans lesquels
R¹ représente un groupe alkyle en C₁-C₂₀, aryle,
R² représente un groupe, aryle,
X représentent tous un atome d'halogène ou un groupe alcoxy en C₁-C₂₀,
n vaut 1, 2 ou 3,
tous les autres symboles et indices ayant la même signification que dans la revendication 3.

5. Utilisation des composés selon les revendications 1 à 4, en tant que substances de marquage visibles ou invisibles.

6. Utilisation selon la revendication 5, les composés selon les revendications 1 à 4 étant utilisés pour le marquage du papier, d'huile minérale, de matières plastiques ou de surfaces métalliques.

7. Utilisation des composés selon les revendications 1 à 4, pour la coloration de matériaux.

8. Utilisation selon la revendication 7, les composés selon les revendications 1 à 4 étant utilisés pour le marquage de matériaux inorganiques et organiques de masse moléculaire élevée.

9. Procédé pour le marquage de matériaux, **caractérisé en ce qu'**on met les matériaux en contact avec des composés selon les revendications 1 à 4.

10. Utilisation des composés selon les revendications 1 à 4, dans la soudure de matériaux au laser.

11. Procédé pour le soudage laser de matériaux, dans lequel on met d'abord les matériaux en contact avec des composés selon les revendications 1 à 4, qui servent d'absorbeurs IR, en option on procède à une augmentation de l'absorption IR par déprotonation et on irradie le matériau avec un laser, dont la longueur d'onde émise recouvre le domaine d'absorption des absorbeurs IR.

12. Utilisation des composés selon les revendications 1 à 4, pour la détection de bases, d'acides ou de variations du pH.

13. Utilisation des composés selon les revendications 1 à 4, en tant qu'adjuvant de dispersion, additif pour pigments pour des pigments organiques et produits intermédiaires pour la production d'additifs pour pigments.

14. Utilisation des composés selon les revendications 1 à 4, dans la gestion de la chaleur ou la gestion de l'énergie.

15. Utilisation des composés selon les revendications 1 à 4, dans la technique photovoltaïque.

16. Utilisation des composés selon les revendications 1 à 4, dans l'enregistrement optique de données.
